(19) Europäisches Patentamt
European Patent Office
Office européen des brevets

(11) **EP 4 607 185 A1**

(12) **DEMANDE DE BREVET EUROPEEN**

(43) Date de publication:
**27.08.2025 Bulletin 2025/35**

(21) Numéro de dépôt: **25159524.5**

(22) Date de dépôt: **21.02.2025**

(51) Classification Internationale des Brevets (IPC):
**G01N 25/18** (2006.01)   **G01N 33/00** (2006.01)
**G01R 21/02** (2006.01)   **G01K 3/14** (2006.01)
**G01K 7/42** (2006.01)   **G01R 21/14** (2006.01)
**G01R 31/08** (2020.01)

(52) Classification Coopérative des Brevets (CPC):
**G01K 7/427; G01K 3/14; G01N 25/18;
G01N 33/004;** G01R 21/02; G01R 21/14;
G01R 31/08; G01R 31/58

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH MA MD TN**

(30) Priorité: **21.02.2024 FR 2401716**

(71) Demandeur: **Nexans
92400 Courbevoie (FR)**

(72) Inventeur: **GUFFOND, Raphaël
69002 Lyon (FR)**

(74) Mandataire: **Cabinet Boettcher
5, rue de Vienne
75008 Paris (FR)**

(54) **SYSTEME DE DETERMINATION D'UNE QUANTITE D'EMISSIONS DE DIOXYDE DE CARBONE RESULTANT DE L'ECHAUFFEMENT D'UN CONDUCTEUR ELECTRIQUE D'UN CABLE ELECTRIQUE PAR EFFET JOULE**

(57)      L'invention concerne un système de détermination (100) d'une quantité d'émissions de dioxyde de carbone résultant de l'échauffement d'un conducteur électrique d'un câble électrique par effet Joule, ledit système de détermination comprenant :
- un câble électrique (10) comprenant au moins un conducteur électrique (12) et au moins une couche de matériau entourant ledit au moins un conducteur,
- une unité de mesure (110) associée au câble électrique, ladite unité de mesure comprenant au moins un capteur de température (20),
- une unité de calcul (120) configurée pour communiquer des informations avec l'unité de mesure, l'unité de calcul étant configurée pour déterminer la température de conducteur $\Theta_{cond}$ au moyen dudit au moins un capteur de température (20), ladite unité de calcul étant en outre configurée pour déterminer une quantité d'émissions de dioxyde de carbone résultant de l'échauffement du conducteur électrique par effet joule en fonction de la température de conducteur $\Theta_{cond}$.

**Fig. 1**

## Description

### Domaine technique

La présente invention concerne un système de détermination d'une quantité d'émissions de dioxyde de carbone résultant de l'échauffement d'un conducteur électrique d'un câble électrique par effet Joule.

Plus spécifiquement, l'invention concerne un système de détermination non-invasif.

### Arrière-plan technologique

Dans un réseau de distribution électrique, un échauffement du conducteur électrique d'un câble électrique se produit par effet Joule lors de la circulation d'un courant le long du conducteur électrique. Avec cette augmentation de température, la résistance du conducteur électrique augmente, ce qui entraîne une augmentation de la perte de puissance.

Cette perte de puissance annuelle a un impact direct sur la quantité des émissions de dioxyde de carbone ($CO_2$).

Il n'existe pas à ce jour de solutions intégrées ou rapportables de détermination de ces émissions de dioxyde de carbone résultant de l'échauffement d'un conducteur électrique d'un câble électrique par effet Joule.

Il existe donc un besoin pour un système de détermination d'une quantité d'émissions de dioxyde de carbone résultant de l'échauffement d'un conducteur électrique d'un câble électrique par effet Joule, directement intégré sur un câble électrique ou rapportable sur celui-ci.

### Résumé de l'invention

Pour cela, l'invention propose un système de détermination d'une quantité d'émissions de dioxyde de carbone résultant de l'échauffement d'un conducteur électrique d'un câble électrique par effet Joule, ledit système de détermination comprenant :

- un câble électrique comprenant au moins un conducteur électrique et au moins une couche de matériau entourant ledit au moins un conducteur,
- une unité de mesure associée au câble électrique, ladite unité de mesure comprenant au moins un capteur de température,
- une unité de calcul configurée pour communiquer des informations avec l'unité de mesure, l'unité de calcul étant configurée pour déterminer la température de conducteur $\Theta_{cond}$ au moyen dudit au moins un capteur de température, ladite unité de calcul étant en outre configurée pour déterminer une quantité d'émissions de dioxyde de carbone résultant de l'échauffement du conducteur électrique par effet joule en fonction de la température de conducteur $\Theta_{cond}$.

Il est apparu que la mesure de température est un moyen particulièrement simple et avantageux pour déterminer la quantité des émissions de $CO_2$ induites par l'échauffement du conducteur électrique. La mesure de températures permet par exemple de déterminer un flux thermique (ou puissance thermique) dont on peut déduire les émissions de $CO_2$.

De préférence, l'unité de calcul est configurée pour déterminer une intensité électrique $I_{cond}$ du conducteur électrique en fonction de la température de conducteur $\Theta_{cond}$. La configuration de l'unité de calcul pour déterminer l'intensité électrique $I_{cond}$ à partir de la température de conducteur $\Theta_{cond}$ permet d'obtenir un système de mesure particulièrement simple à mettre en œuvre et peu coûteux puisqu'il ne comprend pas d'autre capteur que le ou les capteurs de température.

Selon un mode de réalisation du système de détermination, l'unité de calcul est configurée pour déterminer une résistance électrique $R_c$ du conducteur électrique en fonction de la température de conducteur $\Theta_{cond}$.

Cette résistance électrique $R_c$ est notamment déterminée comme suit :

$$R_c = R_0 \times \left(1 + \alpha_{20} \times (\Theta_{cond} - 20)\right) \times \left(1 + y_s + y_p\right)$$

Selon un mode de réalisation du système de détermination, l'unité de calcul est configurée pour déterminer une perte de puissance $P_oL$ en fonction de l'intensité électrique $I_{cond}$ dans le conducteur électrique et de la résistance électrique $R_c$ du conducteur électrique, l'unité de calcul étant configurée pour déterminer la quantité d'émissions de dioxyde de carbone en fonction de ladite perte de puissance $P_oL$.

Cette perte de puissance $P_oL$ est notamment déterminée comme suit :

$$PoL \quad = Rc \times I^2_{cond}$$

Selon un mode de réalisation du système de détermination, celui-ci comprend en outre un boîtier de mesure monté sur le câble électrique, ledit boîtier de mesure comprenant au moins le capteur de température.

Selon un mode de réalisation du système de détermination, le boîtier de mesure comprend en outre l'unité de calcul.

Selon un mode de réalisation du système de détermination, le boîtier de mesure est configuré pour être monté de manière amovible sur le câble électrique.

Selon un mode de réalisation du système de détermination, le boîtier de mesure comprend un dispositif de fixation au câble électrique.

Selon un mode de réalisation du système de détermination, ledit au moins un capteur de température comprend un capteur de température qui est disposé sur une surface externe de ladite au moins une couche de matériau pour mesurer une température périphérique $\Theta_{b1}$ au niveau de la surface externe de ladite au moins une couche de matériau, l'unité de calcul étant configurée pour déterminer la température de conducteur $\Theta_{cond}$ en fonction de la température périphérique $\Theta_{b1}$.

L'utilisation d'un ou plusieurs capteurs de température externes au câble électrique permet une détermination de la température de conducteur $\Theta_{cond}$ non invasive et non-destructrice.

Selon un mode de réalisation du système de détermination, celui-ci comprend en outre :

- une couche additionnelle de matériau disposée autour de ladite au moins une couche de matériau et recouvrant ledit au moins un capteur de température,
- au moins un capteur de température additionnel disposé sur une surface externe de ladite couche de matériau additionnelle pour mesurer une température périphérique additionnelle $\Theta_{b2}$ au niveau de la surface externe de ladite au moins une couche additionnelle de matériau.

Selon un mode de réalisation du système de détermination, l'unité de calcul est configurée pour déterminer la température de conducteur $\Theta_{cond}$ en fonction de la température périphérique $\Theta_{b1}$ et de la température périphérique additionnelle $\Theta_{b2}$.

Ladite au moins une couche de matériau du câble électrique présente une résistance thermique de couche $T_1$.

L'unité de calcul peut comprendre une unité de détermination de la température de conducteur $\Theta_{cond}$. Ainsi, l'unité de détermination est configurée pour déterminer la température de conducteur $\Theta_{cond}$ en fonction de la température périphérique $\Theta_{b1}$ mesurée, la résistance thermique de couche $T_1$ et le flux thermique $W_c$ généré par la circulation d'un courant électrique dans le conducteur électrique.

La détermination de la température de conducteur $\Theta_{cond}$ se fait ici au moyen d'un modèle physique utilisant la température périphérique $\Theta_{b1}$ mesurée, et la résistance thermique de couche $T_1$ et le flux thermique $W_c$ généré par la circulation d'un courant électrique dans le conducteur électrique.

La détermination de l'intensité électrique $I_{cond}$ se fait au moyen de la valeur de température du conducteur $\Theta_{cond}$ et de la connaissance de la géométrie du conducteur associé.

L'utilisation de ce modèle physique permet de s'affranchir de l'utilisation d'une température interne au câble électrique, i.e. mesurée via un composant disposé à proximité du conducteur, ou plus généralement à l'intérieur de la gaine externe du câble électrique.

Le modèle physique utilisé permet d'estimer la température de conducteur $\Theta_{cond}$ au moyen de la température périphérique $\Theta_{b1}$ mesurée, et la résistance thermique de couche $T_1$ et le flux thermique $W_c$ généré par la circulation d'un courant électrique dans le conducteur électrique.

Selon un mode de réalisation du système de détermination, celui-ci comprend en outre :

- au moins une couche additionnelle de matériau disposée autour de ladite au moins une couche de matériau et recouvrant ledit au moins un capteur de température, ladite couche additionnelle de matériau présentant une résistance thermique additionnelle $T_b$,
- au moins un capteur de température additionnel disposé sur une surface externe de ladite couche de matériau additionnelle pour mesurer une température périphérique additionnelle $\Theta_{b2}$ au niveau de la surface externe de ladite au moins une couche additionnelle de matériau.

Selon un mode de réalisation du système de détermination, ladite couche additionnelle de matériau s'étend autour de ladite au moins une couche de matériau uniquement sur une portion de la longueur du câble électrique.

Selon un mode de réalisation du système de détermination, ladite au moins une couche additionnelle de matériau comprend :

- une première portion de couche additionnelle présentant une première résistance thermique additionnelle $T_b$, et
- une deuxième portion de couche additionnelle présentant une deuxième résistance thermique additionnelle $T'_b$, les première $T_b$ et deuxième $T'_b$ résistances thermiques additionnelles étant différentes,

et dans lequel ledit au moins un capteur de température additionnel comprend :

- au moins un premier capteur de température additionnel disposé sur une surface externe de la première portion de couche additionnelle pour mesurer une première température périphérique additionnelle $\Theta_{b2}$,
- au moins un deuxième capteur de température additionnel disposé sur une surface externe de la deuxième portion de couche additionnelle pour mesurer une deuxième température périphérique additionnelle $\Theta'_{b2}$,

l'unité de détermination étant configurée pour déterminer la température de conducteur $\Theta_{cond}$ en outre en fonction de la première $T_b$ et de la deuxième $T'_b$ résistances thermiques additionnelles ainsi que de la première $\Theta_{b2}$ et de la deuxième $\Theta'_{b2}$ températures périphériques additionnelles.

Selon un mode de réalisation du système de détermination, lesdites au moins une première et au moins une deuxième couches additionnelles sont disposées sur des secteurs angulaires différents autour du conducteur électrique.

Lesdites au moins une première et au moins une deuxième couches additionnelles peuvent se recouvrir au moins partiellement.

Selon un mode de réalisation du système de détermination, lesdites au moins une première et au moins une deuxième couches additionnelles forment ensemble une couche s'étendant de manière continue autour du conducteur électrique dans un plan perpendiculaire à l'axe longitudinal d'extension du conducteur électrique.

Selon un mode de réalisation du système de détermination, ledit au moins un capteur de température comprend :

- au moins un premier capteur disposé sur la surface externe de ladite au moins une couche de matériau, entre ladite surface externe et la première portion de couche additionnelle, pour mesurer une température périphérique $\Theta_{b1}$ au niveau de la surface externe de ladite au moins une couche de matériau,
- au moins un deuxième capteur disposé sur la surface externe de ladite au moins une couche de matériau, entre ladite surface externe et la deuxième portion de couche additionnelle, pour mesurer une température périphérique $\Theta'_{b1}$ au niveau de la surface externe de ladite au moins une couche de matériau.

Selon un mode de réalisation du système de détermination, l'unité de détermination est configurée pour déterminer la température de conducteur $\Theta_{cond}$ sur la base de l'équation suivante :

$$\theta_{conducteur} = \frac{(\theta_{b1}T_b - \theta'_{b1}T'_b) \times ((\theta_{b1} - \theta_{b2}))}{T_b(\theta'_{b1} - \theta'_{b2}) - T'_b(\theta_{b1} - \theta_{b2})}$$

$T_b$ étant la première résistance thermique additionnelle de la première couche additionnelle de matériau,
$T'_b$ étant la deuxième résistance thermique additionnelle de la deuxième couche additionnelle de matériau,
$\Theta_{b1}$ est la température périphérique mesurée par ledit au moins un premier capteur de température,
$\Theta_{b2}$ est la température périphérique additionnelle mesurée par ledit au moins un premier capteur de température additionnel,
$\Theta'_{b1}$ est la température périphérique mesurée par ledit au moins un deuxième capteur de température,
$\Theta'_{b2}$ est la température périphérique additionnelle mesurée par ledit au moins un deuxième capteur de température additionnel.

Selon un mode de réalisation du système de détermination, les première et deuxième portions de couche additionnelle sont réalisées dans un matériau différent.

Selon un mode de réalisation du système de détermination, les première et deuxième portions de couche additionnelle présentent une épaisseur différente, prise perpendiculairement à un axe longitudinal d'extension du câble électrique.

Selon un mode de réalisation du système de détermination, ladite au moins une couche de matériau comprend une gaine externe formant ladite surface externe, ledit au moins un capteur de température étant disposé sur ladite gaine externe.

Selon un mode de réalisation du système de détermination, l'unité de détermination est configurée pour déterminer une température de conducteur $\Theta_{cond}$ en fonction de la température périphérique $\Theta_{b1}$ mesurée et la résistance thermique de couche $T_1$ et le flux thermique $W_c$ généré par la circulation d'un courant électrique dans le conducteur électrique.

Selon un mode de réalisation du système de détermination, l'unité de détermination est configurée pour déterminer la température de conducteur $\Theta_{cond}$ sur la base de l'équation suivante :

$$\Theta_{cond} = \Theta_{b1} + W_c*T_1$$

L'unité de détermination peut être disposée à proximité du câble électrique ou à distance de celui-ci.

Selon un mode de réalisation du système de détermination, ladite au moins une couche de matériau comprend une gaine externe formant ladite surface externe, ledit au moins un capteur de température étant disposé sur ladite gaine externe.

Selon un mode de réalisation du système de détermination, celui-ci comprenant en outre :

- une couche additionnelle de matériau disposée autour de ladite au moins une couche de matériau et recouvrant ledit au moins un capteur de température, ladite couche additionnelle de matériau présentant une résistance thermique additionnelle Tb
- au moins un capteur de température additionnel disposé sur une surface externe de ladite couche de matériau additionnelle pour mesurer une température périphérique additionnelle $\Theta b2$ au niveau de la surface externe de ladite au moins une couche additionnelle de matériau.

Selon un mode de réalisation du système de détermination, ladite couche additionnelle de matériau s'étend autour de ladite au moins une couche de matériau uniquement sur une portion de la longueur du câble électrique.

Selon un mode de réalisation du système de détermination, l'unité de détermination est configurée pour déterminer la température de conducteur $\Theta_{cond}$ sur la base de l'équation suivante :

$$\theta_{cond} = \theta_{b1} + \frac{(\theta_{b1} - \theta_{b2})}{T_b} \times T_1$$

$\Theta_{b1}$ est la température périphérique mesurée par ledit au moins un capteur de température,

$\Theta_{b2}$ est la température périphérique additionnelle mesurée par ledit au moins un capteur de température additionnel,

$T_b$ étant la résistance thermique additionnelle de la couche additionnelle de matériau,

$T_1$ étant la résistance thermique de ladite au moins une couche de matériau.

Selon un mode de réalisation du système de détermination, l'unité de détermination est configurée pour déterminer la température de conducteur $\Theta_{cond}$ en outre en fonction d'un échauffement $W_d$ provenant d'une perte diélectrique dans ladite au moins une couche de matériau.

Selon un mode de réalisation du système de détermination, l'unité de détermination est configurée pour déterminer la température de conducteur $\Theta_{cond}$ sur la base de l'équation suivante :

$$\theta_{cond} = \theta_{b1} + T_1 \times \left( W_c - \frac{W_d}{2} \right)$$

$W_d$ étant l'échauffement provenant d'une perte diélectrique dans ladite au moins une couche de matériau.

Selon un mode de réalisation du système de détermination, l'unité de détermination est configurée pour déterminer la température de conducteur $\Theta_{cond}$ sur la base de l'équation suivante :

$$\theta_{cond} = \theta_{b1} + T_1 \times \left( \frac{\theta_{b1} - \theta_{b2}}{T_b} - \frac{W_d}{2} \right)$$

$W_d$ étant l'échauffement provenant d'une perte diélectrique dans ladite au moins une couche de matériau.

Selon un mode de réalisation du système de détermination, ladite au moins une couche additionnelle de matériau et ledit au moins un capteur de température additionnel sont portés par le boîtier de mesure.

Ce boîtier de mesure est de préférence amovible par rapport au câble électrique de manière à réaliser une mesure ponctuelle et localisée sur le câble électrique. Ce boîtier est par exemple un accessoire de mesure.

Le boîtier de mesure présente de préférence une dimension suivant l'axe longitudinal du câble électrique de sorte qu'il s'étend uniquement sur une portion de la longueur du câble électrique.

## Brève description des figures

La description qui va suivre en regard des dessins annexés, donnés à titre d'exemples non limitatifs, fera bien comprendre en quoi consiste l'invention et comment elle peut être réalisée. Sur les figures annexées :

[fig. 1] La figure 1, représente une vue en perspective d'un système de détermination comprenant un câble électrique, une unité de mesure, une unité de calcul pour déterminer une quantité d'émissions de dioxyde de carbone résultant de l'échauffement d'un conducteur électrique du câble électrique ;

[fig. 2] La figure 2 représente une vue en perspective d'un mode de réalisation du système de détermination de la figure 1 comprenant un boîtier de mesure monté sur le câble électrique, le boîtier de mesure comprend notamment l'unité de mesure ;

[fig. 3] La figure 3 représente une vue en coupe du câble électrique selon une première configuration comprenant un conducteur électrique et au moins une couche de matériau ;

[fig. 4] La figure 4 représente une vue en coupe du câble électrique de la figure 3 comprenant une pluralité de capteurs sur une surface externe de ladite au moins une couche de matériau ;

[fig. 5] La figure 5 représente un schéma d'une première modélisation du câble électrique ;

[fig. 6] La figure 6 représente une vue en coupe du câble électrique de la figure 3 dans lequel le système de détermination comprend une couche additionnelle de matériau autour du câble électrique et une pluralité de capteurs additionnels disposés sur une surface externe de cette couche additionnelle de matériau ;

[fig. 7] La figure 7 représente un schéma d'une deuxième modélisation du câble électrique ;

[fig. 8] La figure 8 représente une troisième modélisation du câble électrique dans laquelle les pertes diélectriques dans ladite au moins une couche de matériau sont prises en compte ;

[fig. 9] La figure 9 représente une quatrième modélisation du câble électrique dans laquelle les pertes diélectriques dans ladite au moins une couche de matériau sont prises en compte, selon le deuxième mode de détermination ;

[fig. 10] La figure 10 représente une vue en coupe du câble électrique selon une deuxième configuration comprenant un conducteur électrique, une ou plusieurs couches de matériau entourant le conducteur électrique et un écran entourant lesdites couches de matériau ;

[fig. 11] La figure 11 représente une cinquième modélisation du câble électrique dans laquelle les pertes diélectriques dans ladite au moins une couche de matériau sont prises en compte ainsi que les pertes diélectriques dans l'écran ;

[fig. 12] La figure 12 représente une sixième modélisation du câble électrique pour déterminer la température de conducteur $\Theta_{cond}$ de manière indépendante du milieu environnant ;

[fig. 13] La figure 13 représente une vue en coupe du câble électrique de la figure 6 dans lequel le système de détermination comprend une couche additionnelle de matériau formée d'une première et d'une deuxième portions de couche additionnelle et une pluralité de capteurs additionnels disposés sur une surface externe de chacune des première et deuxième portions de couche additionnelle de matériau ;

[fig. 14] La figure 14 représente un schéma d'une septième modélisation du câble électrique utilisant la première portion de couche additionnelle de la figure 13 ;

[fig. 15] La figure 15 représente un schéma de la septième modélisation du câble électrique utilisant la deuxième portion de couche additionnelle de la figure 13 ;

[fig. 16] La figure 16 représente une équation de détermination de la température de conducteur selon la septième modélisation ;

[fig. 17] La figure 17 représente un schéma d'une huitième modélisation du câble électrique utilisant la première portion de couche additionnelle de la figure 13 ;

[fig. 18] La figure 18 représente un schéma de la huitième modélisation du câble électrique utilisant la deuxième portion de couche additionnelle de la figure 13 ;

[fig. 19] La figure 19 représente une équation de détermination de la température de conducteur selon la huitième modélisation.

## Description de mode(s) de réalisation

Par souci de clarté, les mêmes références désignant les mêmes éléments selon l'état de l'art et selon l'invention sont utilisées pour toutes les figures.

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation. De plus, le terme « comprenant » n'exclut pas d'autres éléments ou étapes.

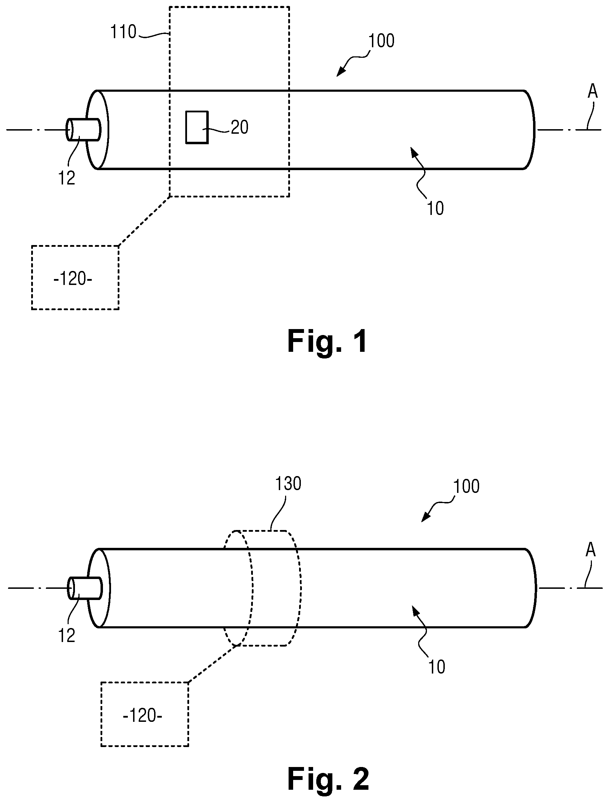

Un système de détermination 100 d'une quantité d'émissions de dioxyde de carbone résultant de l'échauffement d'un conducteur électrique d'un câble électrique par effet Joule est illustré sur la figure 1.

Ce système de détermination 100 comprend un câble électrique 10 comprenant au moins un conducteur électrique 12 et au moins une couche de matériau entourant ledit au moins un conducteur. Cette couche de matériau est par exemple une couche isolante.

Le câble électrique 10 s'étend le long d'un axe longitudinal A.

Ce système de détermination 100 comprend une unité de mesure 110 associée au câble électrique 10. L'unité de mesure comprend au moins un capteur de température 20.

Le système de détermination 100 comprend en outre une unité de calcul 120 configurée pour communiquer des informations avec l'unité de mesure 110.

L'unité de calcul 120 est configurée pour déterminer la température de conducteur $\Theta_{cond}$ et ici également l'intensité électrique $I_{cond}$ du courant électrique circulant dans le conducteur 12 au moyen dudit au moins un capteur de température.

L'unité de calcul 120 est configurée pour déterminer l'intensité électrique $I_{cond}$ en fonction de la température de conducteur $\Theta_{cond}$ et de la géométrie du conducteur 12.

La norme IEC60287 permet d'obtenir la valeur de la résistance d'un conducteur électrique ($R_c$) à partir de sa température et d'au moins un élément de la géométrie du conducteur. Ce ou ces éléments de géométrie comprennent au moins un paramètre parmi : le diamètre du conducteur ; la forme de la section du conducteur ; le nombre de composant(s) (fils par exemple) qui composent le conducteur ; le diamètre de ces composants ; la forme de la section des composants ; la manière dont ces composants sont disposés, etc.

Pour un conducteur donné, on peut donc définir une table, stockée dans une mémoire de l'unité de calcul, qui associe des valeurs de résistance $R_c$ du conducteur 12 à des valeurs de la température $\Theta_{cond}$ du conducteur 12.

La mesure de températures dans un matériau dont la résistance thermique est connue permet de déterminer un flux de transfert thermique ou puissance thermique représentative de la puissance produite par effet Joule. A partir de $P=R_c I_{cond}^2$ on calcule l'intensité $I_{cond}$ du courant circulant dans le conducteur 12.

L'unité de calcul 120 est configurée pour déterminer les émissions de dioxyde de carbone. Il existe des tables fournissant les émissions de dioxyde de carbone en fonction de la puissance thermique. On peut donc stocker dans une mémoire de l'unité de calcul 120 une telle table associant des valeurs d'émissions de dioxyde de carbone à des valeurs de la puissance thermique dégagée par le conducteur 12.

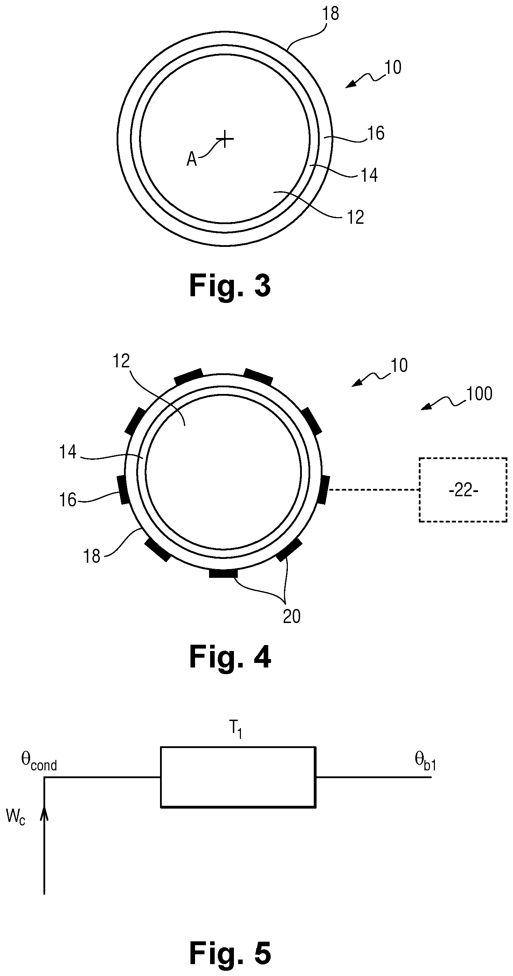

De manière préférée, ledit au moins un capteur de température 20 est disposé à l'extérieur du câble électrique, i.e. sur une surface externe de ce câble électrique 10. Dans cette configuration, la température de conducteur $\Theta_{cond}$ est déterminée au moyen d'un modèle physique décrit ci-après en lien avec les figures 4 à 12.

Dans une variante compatible avec l'invention, ledit au moins un capteur de température peut être disposé à l'intérieur du câble électrique 10. Dans cette variante, la température de conducteur $\Theta_{cond}$ est mesurée directement à proximité du conducteur électrique 12.

L'unité de calcul 120 est en outre configurée pour déterminer une quantité d'émissions de dioxyde de carbone résultant de l'échauffement du conducteur électrique par effet joule en fonction de la température de conducteur $\Theta_{cond}$ et de l'intensité électrique $I_{cond}$ dans le conducteur électrique.

En référence à la figure 2, le système de détermination 100 peut comprendre un boîtier de mesure 130 dans lequel est logé au moins l'un parmi ledit au moins un capteur de température.

L'unité de mesure 110 entière est de préférence portée par le boîtier de mesure 130.

L'unité de calcul 120 est configurée pour être en communication avec l'unité de mesure 110. L'unité de calcul 120 peut être dissociée du boîtier de mesure 130 tel qu'illustré en figure 2 ou bien intégré à ce boîtier de mesure 130.

Ce boîtier de mesure 130 est par exemple monté de manière amovible par rapport au câble électrique 10 de manière à réaliser une mesure ponctuelle et localisée sur le câble électrique 10. Ce boîtier de mesure 130 est par exemple un accessoire de mesure portable.

Le boîtier de mesure 130 présente une dimension suivant l'axe longitudinal A de sorte qu'il s'étend uniquement sur une portion de la longueur du câble électrique 10. Le boîtier de mesure 130 s'étend de préférence autour du câble électrique 10, i.e. autour de l'axe longitudinal A.

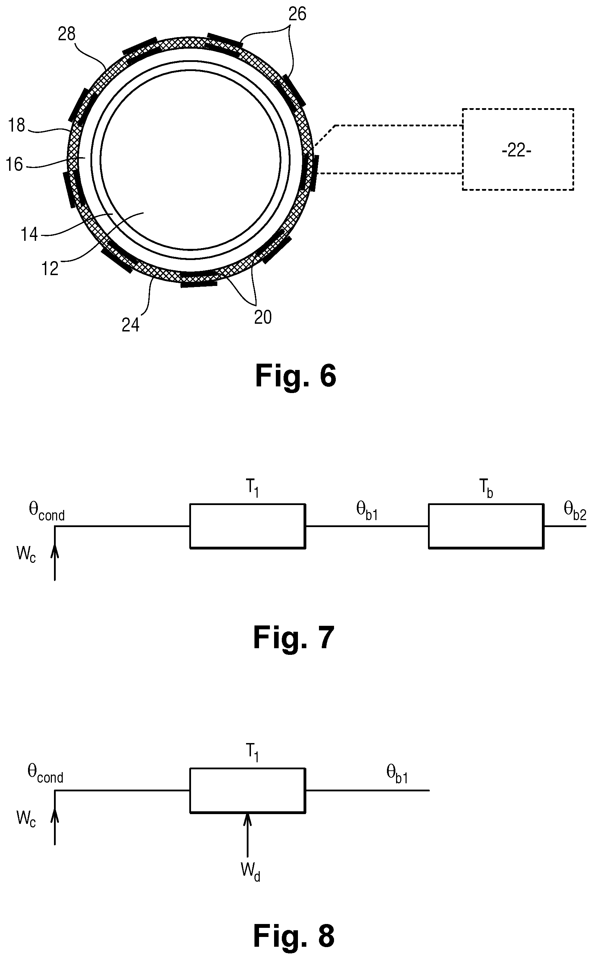

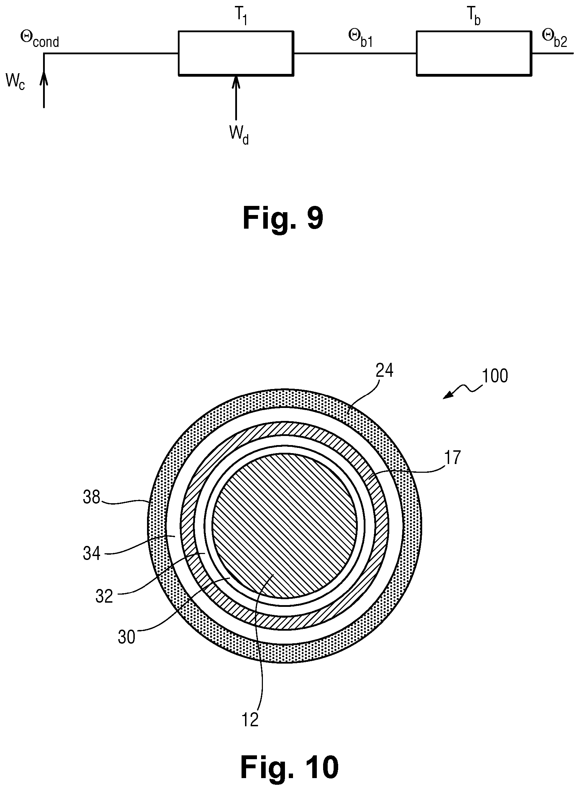

Le boîtier de mesure 130 peut également comprendre une structure additionnelle décrite ci-après, notamment en lien avec les figures 6 et 10.

Pour la détermination des émissions de $CO_2$, l'unité de calcul 120 est configurée pour déterminer une résistance électrique $R_c$ du conducteur électrique en fonction de la température de conducteur $\Theta_{cond}$.

Cette résistance électrique $R_c$ est notamment déterminée comme suit :

$$R_c = R_0 \times \left(1 + \alpha_{20} \times (\Theta_{cond} - 20)\right) \times \left(1 + y_s + y_p\right)$$

avec

$R_0$ étant la résistance en courant continu du conducteur à 20°C, en Ohm,
$Y_s$ étant le facteur d'effet de peau, sans unité,
$Y_p$ étant le facteur d'effet de proximité, sans unité,
$\alpha_{20}$ étant le coefficient de résistivité électrique, en $K^{-1}$ (i.e. par kelvin).

L'unité de calcul 120 est ensuite configurée pour déterminer une perte de puissance $P_oL$ en fonction de l'intensité électrique $I_{cond}$ calculée dans le conducteur électrique et de la résistance électrique $R_c$ du conducteur électrique déterminée.

Cette perte de puissance $P_oL$ est notamment déterminée comme suit :

$$PoL = Rc \times I_{cond}^2$$

L'unité de calcul 120 est configurée pour ensuite déterminer les pertes de puissance annuelles induites par l'échauffement du conducteur électrique 10. La quantité d'émissions de dioxyde de carbone est ensuite déterminée en fonction de ces pertes de puissance annuelles.

Pour déterminer la quantité d'émissions de $CO_2$ en fonction de ces pertes de puissance annuelles, il est possible d'utiliser un facteur d'échelle multiplié par les pertes de puissance annuelles.

D'une manière générale, la quantité d'émissions de $CO_2$ en fonction de ces pertes de puissance annuelles peut être déterminée en utilisant une relation définie par un opérateur de distribution (appelé « Distribution System Operator » en anglais).

En référence à la figure 3, un câble électrique 10 comprend un conducteur électrique 12, une première couche de matériau 14 autour du conducteur électrique 12 et une deuxième couche de matériau 16 autour de la première couche de matériau 14.

Le conducteur électrique 12 s'étend le long d'une axe longitudinal A.

Les première 14 et deuxième 16 couches de matériau s'étendent le long de l'axe longitudinal A, autour du conducteur électrique 12.

La première couche de matériau 14 est par exemple une couche formée d'un matériau isolant électriquement. La première couche de matériau 14 peut donc être considérée comme une couche isolante.

La deuxième couche de matériau 16 forme ici une couche externe du câble électrique 10. La deuxième couche de matériau 16 forme une surface externe 18 du câble électrique 10.

La deuxième couche de matériau 16 est par exemple une gaine externe.

De manière plus générale, le câble électrique 10 peut comporter une ou plusieurs couches de matériau entourant le conducteur électrique 12. Le câble électrique 10 peut notamment comprendre l'un ou plusieurs parmi : un écran, une couche semi-conductrice, une couche isolante, une gaine externe.

Dans une configuration préférée, le câble électrique 10 comprend autour du conducteur, par ordre de disposition du centre vers la périphérie : une couche semi-conductrice, une couche isolante, un écran et une gaine externe. Cette configuration correspond par exemple à un câble électrique configuré pour un réseau de moyenne tension (entre 1 et 52 kV).

Selon un mode de réalisation, le système de détermination 100 est de préférence non-invasif et/ou non destructif. En d'autres termes, le ou les capteurs de température 20 ne sont pas disposés à l'intérieur du câble électrique.

Dans ce mode de réalisation, l'unité de calcul 120 comprend une unité de détermination 22 configurée pour déterminer la température de conducteur $\Theta_{cond}$ au moyen d'un modèle physique utilisant notamment une température périphérique $\Theta_{b1}$ mesurée par ledit au moins un capteur de température 20 disposé sur une surface externe du câble électrique 10.

La détermination de cette température de conducteur $\Theta_{cond}$ au moyen de ce modèle physique est décrite ci-après en lien avec les figures 4 à 12.

Tel qu'illustré sur la figure 4, le système de détermination 100 comprend ledit au moins un capteur de température 20 et une unité de détermination 22.

L'unité de détermination 22 appartient à l'unité de calcul 120.

Le système de détermination 100 peut comprendre une pluralité de capteurs de température 20 répartis autour de l'axe longitudinal A dans un même plan transversal à cet axe longitudinal A. Dans l'exemple de la figure 4, le système de détermination 100 comprend 9 capteurs de température 20.

Le ou les capteurs de température 20 sont configurés pour mesurer une température périphérique $\Theta_{b1}$. Dans cette configuration où les capteurs de température 20 sont disposés au niveau de la surface externe 18 du câble électrique, la température périphérique $\Theta_{b1}$ correspond à la température en surface du câble électrique 10.

Le ou les capteurs de température 20 sont connectés à l'unité de détermination 22 de manière à communiquer la température périphérique $\Theta_{b1}$ à cette unité de détermination 22.

De préférence, les capteurs de température 20 sont équirépartis autour de l'axe longitudinal A. Le système de détermination 100 peut prévoir un ou plusieurs capteurs de température 20 répartis en outre le long de l'axe longitudinal A de manière à mesurer la température périphérique $\Theta_{b1}$ à différentes localisations le long du câble électrique 10.

Cette unité de détermination 22 est configurée pour déterminer la température de conducteur $\Theta_{cond}$, i.e. la température du conducteur électrique 12.

Cette détermination est réalisée de manière non-invasive et non-destructrice. Ainsi, aucun composant n'est inséré sous les couches de matériau ou à proximité du conducteur électrique 12 pour déterminer sa température de conducteur $\Theta_{cond}$. De plus, aucune couche de matériau du câble électrique 10 n'est endommagée ou transpercée pour réaliser cette détermination. Aucun composant tiers n'est intégré à la fabrication du câble électrique 10 telle qu'une fibre optique ou un capteur dans l'une des couches du câble électrique 10 ou bien entre ces couches de matériau.

Ceci permet de réaliser une détermination de la température de conducteur sur un câble électrique existant, par exemple déjà installé in situ, sans avoir la nécessité de l'endommager ou d'y insérer un quelconque outil de mesure.

On considère ici que l'adjonction de composants de mesure ou de couches de matériau supplémentaires n'est pas invasif ou destructif.

L'unité de détermination 22 utilise un modèle physique permettant de déterminer la température de conducteur $\Theta_{cond}$ en fonction de la température périphérique $\Theta_{b1}$ mesurée par le ou les capteurs de température 20.

En référence à la figure 5, la diffusion de la chaleur au travers du câble électrique 10 est représentée sous forme de schéma pour illustrer le modèle physique utilisé par l'unité de détermination 22.

Ce modèle physique repose sur le fait que la diffusion de la chaleur au travers des couches d'un câble électrique suit un comportement proche de celui de la circulation d'un courant au sein d'un circuit électrique comportant une résistance électrique.

Ainsi, le modèle physique établit une relation entre la résistance thermique T1 de ladite au moins une couche de matériau. La résistance thermique $T_1$ peut correspondre à la résistance thermique de l'une ou plusieurs des couches de matériau. Dans l'exemple de la figure 4, la résistance thermique $T_1$ représente la résistance thermique de l'ensemble des première 14 et deuxième 16 couches de matériau. Pour ce modèle physique, les première 14 et deuxième 16 couches de matériau forment donc une seule et même couche de matériau présentant une résistance thermique de couche, appelée $T_1$.

Le passage du courant à l'intérieur du conducteur génère un échauffement induisant un flux thermique $W_c$.

Dans ce modèle physique, la différence de tension $\Delta U$ aux bornes d'une résistance électrique est rapprochée d'une différence de température $\Delta \Theta$ entre les surfaces interne et externe de ladite au moins une couche de matériau (i.e. aux bornes de cette couche de matériau).

Dans l'application du câble électrique 10, les températures aux bornes de la résistance thermique T1 sont d'une part la température du conducteur $\Theta_{cond}$ et d'autre part la température périphérique $\Theta_{b1}$. Ainsi, la différence de température $\Delta \Theta$ s'exprime comme suit : $\Delta \Theta = \Theta_{cond} - \Theta_{b1}$.

Selon ce modèle physique, une relation mathématique est établie entre flux thermique $W_c$, la résistance thermique T1 et la différence de température $\Delta \Theta$ aux bornes de cette résistance thermique. Cette relation est la suivante :

$$\Delta \Theta = T_1 * W_c$$

avec

$\Delta \Theta$ étant la différence de température $\Delta \Theta$ entre les surfaces interne et externe de couche de ladite au moins une couche de matériau,

$T_1$ étant la résistance thermique de ladite au moins une couche de matériau,

$W_c$ étant le flux thermique généré par l'échauffement du conducteur.

La température de conducteur $\Theta_{cond}$ peut donc s'exprimer comme suit :

$$\Theta_{cond} = \Theta_{b1} + W_c{}^*T_1$$

avec

$\Theta_{cond}$ étant la température de conducteur,
$\Theta_{b1}$ étant la température périphérique.

La résistance thermique $T_1$ de ladite au moins une couche de matériau est déterminée comme suit :

$$T_1 = \frac{\rho_T}{2 \times \pi} \ln(1 + 2 * \frac{t_1}{d_c})$$

avec

$\rho_T$ étant la conductivité thermique de ladite au moins une couche de matériau,
$d_c$ étant le diamètre interne de ladite au moins une couche de matériau,
$t_1$ étant l'épaisseur de ladite au moins une couche de matériau.

Le système de détermination 100 comprend au moins une couche additionnelle 24 de matériau et au moins un capteur de température additionnel 26.

Ce mode de détermination permet de s'affranchir de la mesure de l'intensité électrique $I_{cond}$ du courant circulant dans le conducteur.

Ladite au moins une couche additionnelle de matériau 24 est disposée autour de ladite au moins une couche de matériau. La ou les couches additionnelles de matériau 24 recouvre ledit au moins un capteur de température 22, tel que visible sur la figure 6.

Ce mode de détermination peut être utilisé pour la détermination de la température de conducteur $\Theta_{cond}$ selon différentes modélisations d'un câble électrique 10. Ces différentes modélisations peuvent impliquer différentes hypothèses (e.g. prise en compte ou non de pertes diélectriques) ou bien différentes configurations du câble électrique 10.

L'unité de détermination 22 est configurée pour déterminer la température de conducteur $\Theta_{cond}$ selon l'une ou plusieurs modélisations, notamment l'une ou plusieurs parmi les modélisations présentées ci-après.

**Câble électrique sans écran et sans prise en compte des pertes diélectriques**

L'unité de détermination 22 est configurée pour déterminer la température de conducteur $\Theta_{cond}$ selon une première et une deuxième modélisations respectivement illustrées sur les figures 5 et 7.

Dans les première et deuxième modélisations, les pertes diélectriques dans ladite au moins une couche de matériau ne sont pas prises en compte ou considérées comme minimes.

Dans ces première et deuxième modélisations, le câble électrique 10 est dépourvu d'écran.

La première modélisation s'applique à câble électrique 10 comprenant un conducteur électrique 12 et une ou plusieurs couches de matériau entourant le conducteur électrique 12. Un ou plusieurs capteurs de température 20 sont disposés sur la surface externe 18 de ladite au moins une couche de matériau.

Le câble électrique 10 selon la figure 4 est un exemple compatible avec cette première modélisation.

L'unité de détermination 100 comprend une structure additionnelle illustrée en figure 6. Ainsi, le système de détermination 100 comprend au moins une couche additionnelle 24 de matériau et au moins un capteur de température additionnel 26.

Ladite au moins une couche additionnelle de matériau 24 présente une résistance thermique additionnelle $T_b$.

Ladite au moins une couche additionnelle de matériau 24 est par exemple au moins une couche isolante électriquement.

Le matériau de ladite au moins une couche additionnelle de matériau 24 présente de préférence une résistance thermique entre 0,001 m$^2$.K/W et 0,1 m$^2$.K/W. Dans cette plage de résistance thermique, ladite au moins une couche de matériau 24 permet d'éviter la surchauffe du conducteur tout permettant une différence de température suffisamment grande pour être mesurée.

Ledit au moins un capteur de température additionnel 26 permet de mesurer une température périphérique additionnelle $\Theta_{b2}$ au niveau de la surface externe 28 de ladite au moins une couche additionnelle de matériau 24.

Le système de détermination 100 peut comprendre une pluralité de capteurs de température additionnels 26 répartis autour de l'axe longitudinal A dans un même plan transversal à l'axe longitudinal A. Dans l'exemple de la figure 6, le système de détermination 100 comprend 9 capteurs de température additionnels 26.

Le ou les capteurs de température additionnels 26 sont connectés à l'unité de détermination 22 de manière à communiquer la température périphérique additionnelle $\Theta_{b2}$ à cette unité de détermination 22.

De préférence, les capteurs de température additionnels 26 sont équirépartis autour de l'axe longitudinal A. Le système de détermination 100 peut prévoir un ou plusieurs capteurs de température additionnels 26 répartis en outre le long de l'axe longitudinal A de manière à mesurer la température périphérique additionnelle $\Theta_{b2}$ à différentes localisations le long du câble électrique 10.

De préférence, le nombre et/ou la position angulaire et/ou la position longitudinale des capteurs de température 20 sont respectivement identiques au nombre et/ou à la position angulaire et/ou la position longitudinale des capteurs de température additionnels 26.

Le câble électrique 10 équipé de ladite au moins une couche additionnelle de matériau 24 et dudit au moins un capteur de température additionnel 26 est modélisé par une deuxième modélisation sur la figure 7. Ladite au moins une couche additionnelle de matériau 24 est considérée comme une résistance de valeur $T_b$ en série avec la résistance de valeur $T_1$ correspondant à ladite au moins une couche de matériau.

On exprime le flux thermique $W_c$ commet suit :

$$W_c = \frac{(\Theta_{b1} - \Theta_{b2})}{T_b}$$

La température de conducteur peut ainsi être exprimée comme suit :

$$\theta_{cond} = \theta_{b1} + \frac{(\theta_{b1} - \theta_{b2})}{T_b} \times T_1$$

La détermination de la température de conducteur $\Theta_{cond}$ peut ainsi être déterminée sans nécessiter la valeur de l'intensité du courant circulant dans le conducteur électrique 12. Cette détermination est rendue possible par l'adjonction d'une couche additionnelle et d'un capteur additionnel.

**Câble électrique sans écran et avec prise en compte des pertes diélectriques**

L'unité de détermination 22 est configurée pour déterminer la température de conducteur $\Theta_{cond}$ selon une troisième et une quatrième modélisations respectivement illustrées sur les figures 8 et 9.

.

Dans les troisième et quatrième modélisations, les pertes diélectriques dans ladite au moins une couche de matériau sont prises en compte.

Dans ces troisième et quatrième modélisations, le câble électrique 10 est dépourvu d'écran.

Dans ces troisième et quatrième modélisations, les pertes diélectriques dans ladite au moins une couche de matériau sont considérées comme un flux thermique de perte $W_d$ au niveau de la résistance de valeur $T_1$ correspondant à ladite au moins une couche de matériau. Ce flux thermique de perte $W_d$ est visible sur les figures 8 et 9.

La troisième modélisation s'applique à un câble électrique 10 comprenant un conducteur électrique 12 et une ou plusieurs couches de matériau entourant le conducteur électrique 12. Un ou plusieurs capteurs de température 20 sont disposés sur la surface externe 18 de ladite au moins une couche de matériau.

Le câble électrique 10 selon la figure 4 est un exemple compatible avec cette troisième modélisation.

L'unité de détermination 100 comprend une structure additionnelle telle qu'illustrée en figure 6. Ainsi, le système de détermination 100 comprend au moins une couche additionnelle 24 de matériau et au moins un capteur de température additionnel 26.

Le câble électrique 10 équipé de ladite au moins une couche additionnelle de matériau 24 et dudit au moins un capteur de température additionnel 26 est modélisé par une quatrième modélisation sur la figure 9.

Ladite au moins une couche additionnelle de matériau 24 est considérée comme une résistance de valeur $T_b$ en série avec la résistance de valeur $T_1$ correspondant à ladite au moins une couche de matériau.

On exprime la température de conducteur $\Theta_{cond}$ comme suit :

$$\theta_{cond} = \theta_{b1} + T_1 \times \left( \frac{(\theta_{b1} - \theta_{b2})}{T_b} - \frac{W_d}{2} \right)$$

L'équation précédente est obtenue en considérant les équations suivantes :

$$\theta_{\mathrm{cond}} = \theta_{b1} + T_1 \times \left(W_c + \frac{W_d}{2}\right)$$

et

$$\theta_{\mathrm{b1}} = \theta_{b2} + T_b \times (W_d + W_c)$$

Le flux thermique de perte $W_d$ est déterminé en fonction de la tension appliquée au conducteur électrique 12, de la fréquence de la tension appliquée au conducteur électrique 12 et des caractéristiques diélectriques de ladite au moins une couche de matériau.

**Câble électrique avec écran et prise en compte des pertes diélectriques**

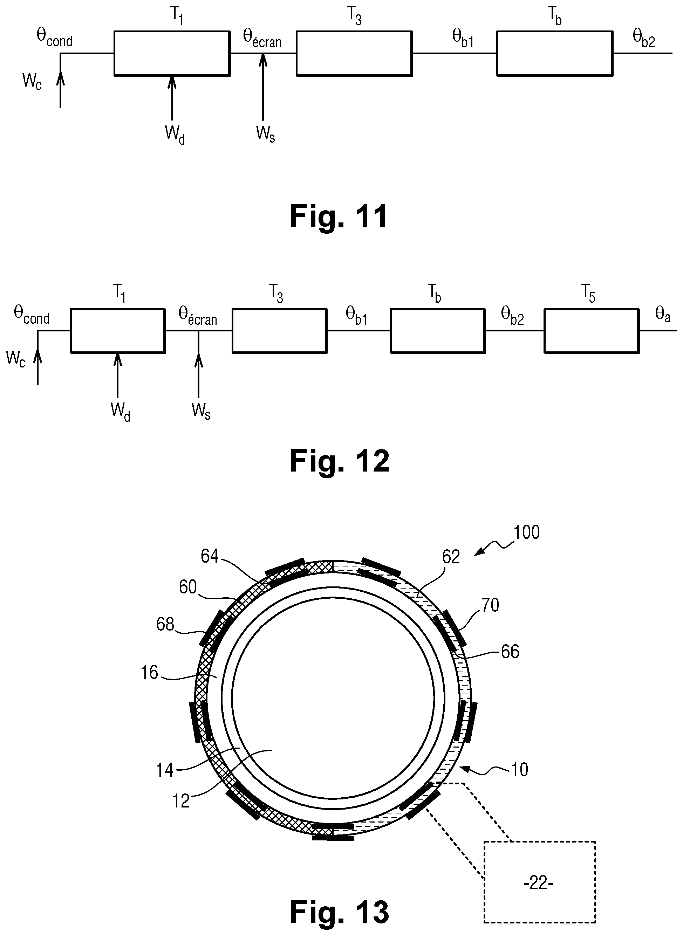

L'unité de détermination 22 est en outre configurée pour déterminer la température de conducteur $\Theta_{\mathrm{cond}}$ dans une configuration du câble électrique 10 comprenant un écran 17.
Tel qu'illustré en figure 10, le câble électrique 10 comprend un conducteur électrique 12, une ou plusieurs couches de matériau entourant le conducteur électrique 12 et un écran 17 entourant lesdites couches de matériau.
Lesdites couches de matériau sont par exemple une couche diélectrique 30 entourant le conducteur électrique 12 et une couche isolante 32 disposée entre la couche diélectrique 30 et l'écran 17.
Le câble électrique 10 comprend également une couche externe 34, par exemple une gaine externe, définissant une surface externe 38 de la couche externe 34. La couche externe 34 peut comprendre une pluralité de couches de matériau.
La couche externe 34 présente une résistance thermique $T_3$.
Un ou plusieurs capteurs de température 20 sont disposés sur la surface externe 38 de la couche externe 34.
Des pertes dans l'écran 17 sont modélisées par un flux thermique d'écran $W_s$. Ces pertes sont dues à un échauffement par effet joule dans l'écran 17.
La détermination du flux thermique d'écran $W_s$ requiert une mesure invasive sur le câble électrique 10. Pour s'affranchir d'exprimer la température de conducteur $\Theta_{\mathrm{cond}}$ en fonction du flux thermique $W_s$, il est prévu ici d'exprimer la température de conducteur $\Theta_{\mathrm{cond}}$ en fonction de l'intensité $I_{\mathrm{cond}}$ du courant circulant dans le conducteur électrique 12 et d'utiliser une structure additionnelle comprenant au moins une couche additionnelle de matériau 24 et au moins un capteur de température additionnel 26, tel que visible sur la figure 10.
Ladite au moins une couche additionnelle de matériau 24 présente une résistance thermique $T_b$.
Une cinquième modélisation est illustrée à la figure 11 prenant en compte les pertes d'écran (flux thermique $W_s$) ainsi que les pertes diélectriques dans ladite au moins une couche (flux thermique $W_d$) et comprenant trois résistances en série pour modéliser les résistances thermiques de ladite au moins une couche de matériau ($T_1$), de la gaine externe 34 ($T_3$) et de ladite au moins une couche additionnelle de matériau 24 ($T_b$).
Dans cette cinquième modélisation, la température de conducteur $\Theta_{\mathrm{cond}}$ s'exprime comme suit :

$$\theta_{cond} = \frac{\theta_{b1} + T_3 \dfrac{\Delta\theta_b}{T_b} + \dfrac{1}{2} T_1 W_d - (R_0 I^2) T_1 (1 - 20 \times \alpha_{20})}{1 - R_0 I_{cond}^2 \alpha_{20} T_1}$$

$\Theta_{b1}$ étant la température périphérique mesurée par ledit au moins un capteur de température 20,
$\Theta_{b2}$ étant la température périphérique additionnelle mesurée par ledit au moins un capteur de température additionnel 26,
$\Delta\Theta$ étant la différence de température $\Theta_{b1}$ - $\Theta_{b2}$,
$T_1$ étant la résistance thermique de ladite au moins une couche de matériau,
$T_b$ étant la résistance thermique de ladite au moins une couche additionnelle de matériau 24,
$T_3$ étant la résistance thermique de couche externe 34,
$R_0$ étant la résistance en courant continu du conducteur à 20°C, en Ohm,
$Y_s$ étant le facteur d'effet de peau, sans unité,
$Y_p$ étant le facteur d'effet de proximité, sans unité,
$\alpha_{20}$ étant le coefficient de résistivité électrique, en K$^{-1}$ (i.e. par kelvin).

L'unité de détermination 22 est ainsi capable de déterminer la température de conducteur $\Theta_{\mathrm{cond}}$ indépendamment du flux thermique d'écran $W_s$.

Cette expression de la température de conducteur $\Theta_{cond}$ est obtenue en considérant que :

$$\theta_{cond} = \theta_{surf} + n(W_c + W_s + W_d)T_3 + \left(W_c + \frac{W_d}{2}\right)T_1$$

$$n(W_c + W_s + W_d) = \frac{\Delta\theta_b}{T_b}$$

$$\theta_{conducteur} = \theta_{b1} + T_3\frac{\Delta\theta_b}{T_b} + \left(W_c + \frac{W_d}{2}\right)T_1$$

avec $\Theta_{surf}$ étant la température périphérique au niveau de la surface externe du câble électrique 10, et n étant le nombre de conducteurs électriques 12.

Tel que détaillé précédemment, la résistance thermique $T_1$ est déterminée comme suit :

$$T_1 = \frac{\rho_T}{2\times\pi}\ln(1 + 2*\frac{t_1}{d_c})$$

La résistance thermique $T_3$ de la couche externe 34 est déterminée comme suit :

$$T_3 = \frac{\rho_T}{2\times\pi}\ln(1 + 2*\frac{t_3}{D_a})$$

avec

$t_3$ étant l'épaisseur de la couche externe 34,
$D_a$ étant le diamètre interne de la couche externe 34.

Selon une sixième modélisation illustrée en figure 12, l'unité de détermination est également configurée pour déterminer la température de conducteur $\Theta_{cond}$ de manière indépendante du milieu environnant, notamment de la température de ce milieu environnant.
Cette sixième modélisation s'applique à la même configuration de câble électrique 10 que la cinquième modélisation. En d'autres termes, la sixième modélisation s'applique à un câble électrique du type de celui de la figure 10 avec une structure additionnelle et un écran 17.
Selon le milieu environnant, la chaleur va être plus ou moins bien évacuée du câble électrique 10. Le milieu environnant est modélisé par une couche de matériau avec une certain résistance thermique $T_5$ et une température $\theta_a$ correspondant à la température ambiante du milieu environnant.
La température de conducteur $\Theta_{cond}$ peut s'exprimer ainsi :

$$\theta_{cond} = \theta_a + \left(W_c + \frac{W_d}{2}\right)T_1 + n(W_c + W_d + W_s)T_3 + n(W_c + W_d + W_s)T_b + n(W_c + W_d + W_s)T_5$$

La différence de température $\Theta_{b1} - \Theta_{b2}$ de part et d'autre de la couche additionnelle de matériau 24 permet d'exprimer la température de conducteur $\Theta_{cond}$ comme suit :

$$\theta_{cond} = \theta_{b1} + \left(W_c + \frac{W_d}{2}\right)T_1 + n(W_c + W_d + W_s)T_b$$

et

$$\theta_{b1} - \theta_{b2} = n(W_c + W_d + W_s)T_b$$

La température de conducteur $\Theta_{cond}$ peut ainsi être déterminée par l'unité de détermination 22 indépendamment du milieu environnant.

En lien avec les figures 13 à 19, l'unité de détermination 22 est en outre configurée pour déterminer la température de conducteur $\Theta_{cond}$ selon une septième et une huitième modélisations.

Dans les septième et huitième modélisations, l'unité de détermination 22 est configurée pour déterminer la température de conducteur $\Theta_{cond}$ sans nécessiter de détermination du flux thermique $W_c$ généré par la circulation d'un courant électrique dans le conducteur électrique.

Pour ces septième et huitième modélisations, le système de détermination 100 est semblable à celui de la figure 6 avec pour différence que ladite au moins une couche additionnelle 24 comprend une première 60 et une deuxième 62 portions de couche additionnelle, tel qu'illustré en figure 13.

La première portion de couche additionnelle 60 présente une première résistance thermique additionnelle $T_b$ La deuxième portion de couche additionnelle 62 présente une deuxième résistance thermique additionnelle $T'_b$. Les première $T_b$ et deuxième $T'_b$ résistances thermiques additionnelles sont différentes.

Cette différence entre les première $T_b$ et deuxième $T'_b$ résistances thermiques additionnelles peut être obtenue par utilisation d'un matériau différent et/ou d'une ou plusieurs caractéristiques géométriques différentes entre les première 60 et deuxième 62 portions de couche additionnelle. Un exemple de caractéristique géométrique différente est une épaisseur différente, prise le long d'un axe perpendiculaire à l'axe longitudinal d'extension A du conducteur électrique.

Cette différence entre les première $T_b$ et deuxième $T'_b$ résistances thermiques additionnelles permet de construire deux équations différentes ayant pour inconnue la température de conducteur $\Theta_{cond}$. Il est ainsi possible de s'affranchir de la connaissance du flux thermique $W_c$.

Une pluralité de premiers capteurs de température 64 sont disposés sur la surface externe 18 de ladite au moins une couche de matériau, entre ladite surface externe 18 et la première portion de couche additionnelle 60. La pluralité de premiers capteurs de température 64 permettent de mesurer une première température périphérique $\Theta_{b1}$ au niveau de la surface externe 18 de ladite au moins une couche de matériau.

Une pluralité de deuxièmes capteurs de température 66 sont disposés sur la surface externe 18 de ladite au moins une couche de matériau, entre ladite surface externe 18 et la deuxième portion de couche additionnelle 62. La pluralité de deuxièmes capteurs de température 66 permettent de mesurer une deuxième température périphérique $\Theta'_{b1}$ au niveau de la surface externe 18 de ladite au moins une couche de matériau.

Une pluralité de premiers capteurs de température additionnels 68 sont disposés sur une surface externe de la première portion de couche additionnelle 60 pour mesurer une première température périphérique additionnelle $\Theta_{b2}$.

Une pluralité de deuxièmes capteurs de température additionnels sont disposés sur une surface externe de la deuxième portion de couche additionnelle pour mesurer une deuxième température périphérique additionnelle $\Theta'_{b2}$.

L'unité de détermination 22 est configurée pour déterminer la température de conducteur $\Theta_{cond}$ en outre en fonction de la première $T_b$ et de la deuxième $T'_b$ résistances thermiques additionnelles ainsi que de la première $\Theta_{b2}$ et de la deuxième $\Theta'_{b2}$ températures périphériques additionnelles.

**Câble électrique sans écran sans prise en compte des pertes diélectriques**

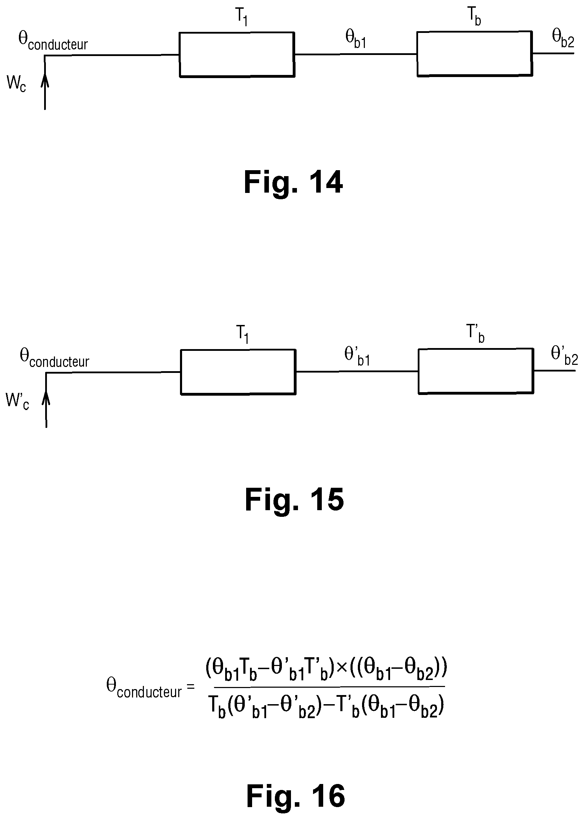

La septième modélisation est illustrée dans les figures 14 à 16.

Dans la septième modélisation, la température de conducteur $\Theta_{cond}$ s'exprime comme ci-après :

$$\theta_{conducteur} = \frac{(\theta_{b1}T_b - \theta'_{b1}T'_b) \times ((\theta_{b1} - \theta_{b2}))}{T_b(\theta'_{b1} - \theta'_{b2}) - T'_b(\theta_{b1} - \theta_{b2})}$$

Cette équation est obtenue via les développements suivant :

$$\frac{(\theta_{conducteur} - \theta_{b1})}{T_1} = \frac{(\theta_{b1} - \theta_{b2})}{T_b}$$

$$\frac{(\theta_{conducteur} - \theta'_{b1})}{T_1} = \frac{(\theta'_{b1} - \theta'_{b2})}{T'_b}$$

$$(\theta_{conducteur} - \theta_{b1}) \frac{T_b}{(\theta_{b1} - \theta_{b2})} = (\theta_{conducteur} - \theta'_{b1}) \frac{T'_b}{(\theta'_{b1} - \theta'_{b2})}$$

$$\theta_{conducteur} = \frac{\theta_{b1} T_b - \theta'_{b1} T'_b}{(\theta'_{b1} - \theta'_{b2})} / \left( \frac{T_b}{(\theta_{b1} - \theta_{b2})} - \frac{T'_b}{(\theta'_{b1} - \theta'_{b2})} \right)$$

**Câble électrique sans écran avec prise en compte des pertes diélectriques**

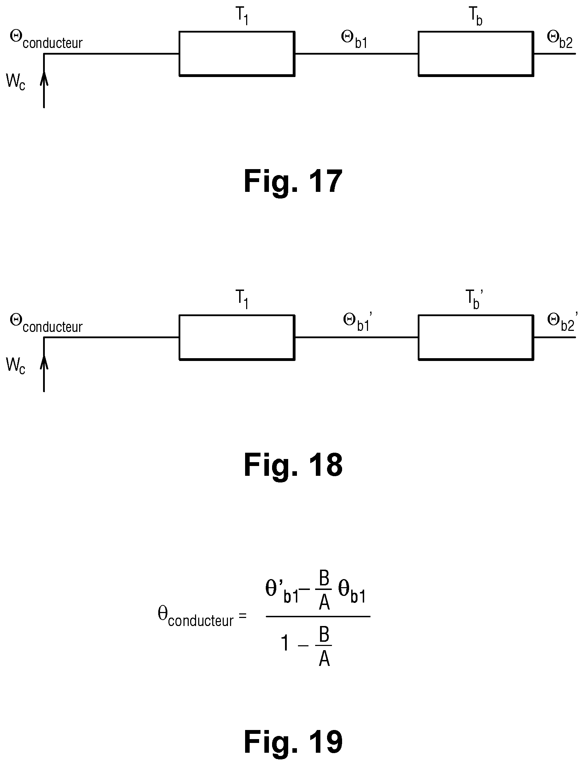

La huitième modélisation est illustrée dans les figures 17 à 19.

Dans la huitième modélisation, la température de conducteur $\Theta_{cond}$ s'exprime comme ci-après:

$$\theta_{conducteur} = \frac{\theta'_{b1} - \frac{B}{A} \theta_{b1}}{1 - \frac{B}{A}}$$

Cette équation est obtenue via les développements suivant :

$$\theta_{conducteur} = \theta_{b1} + T_1 \times \left( \frac{(\theta_{b1} - \theta_{b2})}{T_b} - \frac{W_d}{2} \right)$$

$$\theta_{conducteur} = \theta_{b1}' + T_1 \times \left( \frac{(\theta_{b1}' - \theta_{b2}')}{T_b'} - \frac{W_d}{2} \right)$$

$$A = \left( \frac{(\theta_{b1} - \theta_{b2})}{T_b} - \frac{W_d}{2} \right)$$

$$B = \left( \frac{(\theta_{b1}' - \theta_{b2}')}{T_b'} - \frac{W_d}{2} \right)$$

$\Theta_{b1}'$ étant la première température périphérique au niveau de la surface externe 18 de ladite au moins une couche de matériau,

$\Theta_{b2}'$ étant la deuxième température périphérique additionnelle,

$T_b'$ étant la deuxième résistance thermique additionnelle.

Bien entendu, l'invention n'est pas limitée aux modes de réa-lisation décrits mais englobe toute variante entrant dans le champ de l'invention telle que définie par les revendications.

En particulier, on comprend que, s'il est avantageux de pouvoir calculer l'intensité du courant circulant dans le conducteur 12 à partir de la mesure de température (sans besoin d'un capteur de courant dédié), ce calcul n'est pas nécessaire pour déterminer les émissions de dioxyde de carbone. Les émissions de dioxyde de carbone peuvent être déterminées uniquement à partir des mesures de températures par le calcul de la puissance thermique (ou flux thermique) produite par effet Joule par le courant circulant dans le conducteur 12. Il est indiqué qu'on utilise à cette fin des tables mettant en relation les émissions de dioxyde de carbone et la puissance thermique, mais il est possible alternativement d'utiliser un modèle numérique par exemple élaboré de manière empirique.

Le système peut comprendre un ou plusieurs capteurs de température.

**Revendications**

1. Système de détermination (100) d'une quantité d'émissions de dioxyde de carbone résultant de l'échauffement d'un conducteur électrique d'un câble électrique par effet Joule, ledit système de détermination comprenant :

- un câble électrique (10) comprenant au moins un conducteur électrique (12) et au moins une couche de matériau (14, 16, 17, 30, 32, 34) entourant ledit au moins un conducteur,
- une unité de mesure (110) associée au câble électrique, ladite unité de mesure comprenant au moins un capteur de température (20),
- une unité de calcul (120) configurée pour communiquer des informations avec l'unité de mesure, l'unité de calcul étant configurée pour déterminer la température de conducteur $\Theta_{cond}$ au moyen dudit au moins un capteur de température (20), ladite unité de calcul étant en outre configurée pour déterminer une quantité d'émissions de dioxyde de carbone résultant de l'échauffement du conducteur électrique par effet joule en fonction de la température de conducteur $\Theta_{cond}$.

2. Système de détermination (100) selon la revendication 1, dans lequel l'unité de calcul est configurée pour déterminer une intensité électrique $I_{cond}$ du conducteur électrique en fonction de la température de conducteur $\Theta_{cond}$.

3. Système de détermination (100) selon la revendication 1 ou 2, dans lequel l'unité de calcul est configurée pour déterminer une résistance électrique $R_c$ du conducteur électrique en fonction de la température de conducteur $\Theta_{cond}$.

4. Système de détermination (100) selon la revendication 3, dans lequel l'unité de calcul est configurée pour déterminer une perte de puissance $P_oL$ en fonction de l'intensité électrique $I_{cond}$ dans le conducteur électrique et de la résistance électrique $R_c$ du conducteur électrique, l'unité de calcul étant configurée pour déterminer la quantité d'émissions de dioxyde de carbone en fonction de ladite perte de puissance $P_oL$.

5. Système de détermination (100) selon l'une quelconque des revendications précédentes, comprenant en outre un boîtier de mesure monté sur le câble électrique, ledit boîtier de mesure comprenant au moins ledit au moins un capteur de température.

6. Système de détermination (100) selon la revendication 5, dans lequel le boîtier de mesure comprend en outre l'unité de calcul.

7. Système de détermination (100) selon la revendication 5 ou 6, dans lequel le boîtier de mesure est configuré pour être monté de manière amovible sur le câble électrique.

8. Système de détermination (100) selon la revendication 7, dans lequel le boîtier de mesure comprend un dispositif de fixation au câble électrique.

9. Système de détermination (100) selon l'une quelconque des revendications précédentes, dans lequel ledit au moins un capteur de température comprend un capteur de température qui est disposé sur une surface externe de ladite au moins une couche de matériau pour mesurer une température périphérique $\Theta_{b1}$ au niveau de la surface externe de ladite au moins une couche de matériau, l'unité de calcul étant configurée pour déterminer la température de conducteur $\Theta_{cond}$ en fonction de la température périphérique $\Theta_{b1}$.

10. Système de détermination (100) selon la revendication 9, comprenant en outre :

- une couche additionnelle de matériau disposée autour de ladite au moins une couche de matériau et recouvrant ledit au moins un capteur de température,
- au moins un capteur de température additionnel disposé sur une surface externe de ladite couche de matériau additionnelle pour mesurer une température périphérique additionnelle $\Theta_{b2}$ au niveau de la surface externe de ladite au moins une couche additionnelle de matériau.

11. Système de détermination (100) selon la revendication précédente, dans lequel l'unité de calcul est configurée pour déterminer la température de conducteur $\Theta_{cond}$ en fonction de la température périphérique $\Theta_{b1}$ et de la température périphérique additionnelle $\Theta_{b2}$.

12. Système de détermination (100) selon l'une des revendications précédentes, dans lequel l'unité de calcul est configurée pour déterminer l'intensité électrique $I_{cond}$ en fonction de la température de conducteur $\Theta_{cond}$ et de la géométrie du conducteur.

**13.** Système de détermination (100) selon la revendication 10 ou 11, dans lequel ladite au moins une couche additionnelle de matériau comprend :

- une première portion de couche additionnelle présentant une première résistance thermique additionnelle $T_b$, et
- une deuxième portion de couche additionnelle présentant une deuxième résistance thermique additionnelle $T'_b$, les première $T_b$ et deuxième $T'_b$ résistances thermiques additionnelles étant différentes,

et dans lequel ledit au moins un capteur de température additionnel comprend :

- au moins un premier capteur de température additionnel disposé sur une surface externe de la première portion de couche additionnelle pour mesurer une première température périphérique additionnelle $\Theta_{b2}$,
- au moins un deuxième capteur de température additionnel disposé sur une surface externe de la deuxième portion de couche additionnelle pour mesurer une deuxième température périphérique additionnelle $\Theta'_{b2}$,

l'unité de détermination étant configurée pour déterminer la température de conducteur $\Theta_{cond}$ en outre en fonction de la première $T_b$ et de la deuxième $T'_b$ résistances thermiques additionnelles ainsi que de la première $\Theta_{b2}$ et de la deuxième $\Theta'_{b2}$ températures périphériques additionnelles.

**14.** Système de détermination (100) selon la revendication précédente, dans lequel lesdites au moins une première et au moins une deuxième couches additionnelles sont disposées sur des secteurs angulaires différents autour du conducteur électrique.

**15.** Système de détermination (100) selon la revendication précédente, dans lequel ledit au moins un capteur de température (20) comprend :

- au moins un premier capteur disposé sur la surface externe (18) de ladite au moins une couche de matériau, entre ladite surface externe (18) et la première portion de couche additionnelle, pour mesurer une température périphérique $\Theta_{b1}$ au niveau de la surface externe de ladite au moins une couche de matériau,
- au moins un deuxième capteur disposé sur la surface externe (18) de ladite au moins une couche de matériau, entre ladite surface externe (18) et la deuxième portion de couche additionnelle, pour mesurer une température périphérique $\Theta'_{b1}$ au niveau de la surface externe de ladite au moins une couche de matériau.

**16.** Système de détermination (100) selon la revendication précédente, dans lequel l'unité de détermination est configurée pour déterminer la température de conducteur $\Theta_{cond}$ sur la base de l'équation suivante :

$$\theta_{conducteur} = \frac{(\theta_{b1}T_b - \theta'_{b1}T'_b) \times ((\theta_{b1} - \theta_{b2}))}{T_b(\theta'_{b1} - \theta'_{b2}) - T'_b(\theta_{b1} - \theta_{b2})}$$

$T_b$ étant la première résistance thermique additionnelle de la première couche additionnelle de matériau,
$T'_b$ étant la deuxième résistance thermique additionnelle de la deuxième couche additionnelle de matériau,
$\Theta_{b1}$ est la température périphérique mesurée par ledit au moins un premier capteur de température,
$\Theta_{b2}$ est la température périphérique additionnelle mesurée par ledit au moins un premier capteur de température additionnel,
$\Theta'_{b1}$ est la température périphérique mesurée par ledit au moins un deuxième capteur de température,
$\Theta'_{b2}$ est la température périphérique additionnelle mesurée par ledit au moins un deuxième capteur de température additionnel.

**17.** Système de détermination (100) selon l'une quelconque des revendications 12 à 15, dans lequel les première et deuxième portions de couche additionnelle sont réalisées dans un matériau différent.

**18.** Système de détermination (100) selon l'une quelconque des revendications 12 à 16, dans lequel les première et deuxième portions de couche additionnelle présentent une épaisseur différente, prise perpendiculairement à un axe longitudinal d'extension du câble électrique (10).

110

100

12

20

A

10

-120-

## Fig. 1

130

100

12

A

10

-120-

## Fig. 2

18

10

16

14

12

A +

**Fig. 3**

12

14

16

18

10

100

-22-

20

**Fig. 4**

$T_1$

$\theta_{cond}$

$\theta_{b1}$

$W_c$

**Fig. 5**

**Fig. 6**

**Fig. 7**

**Fig. 8**

**Fig. 9**

**Fig. 10**

**Fig. 11**

**Fig. 12**

**Fig. 13**

$\theta_{conducteur}$      $T_1$      $\theta_{b1}$      $T_b$      $\theta_{b2}$

$W_c$

## Fig. 14

$\theta_{conducteur}$      $T_1$      $\theta'_{b1}$      $T'_b$      $\theta'_{b2}$

$W'_c$

## Fig. 15

$$\theta_{conducteur} = \frac{(\theta_{b1}T_b - \theta'_{b1}T'_b) \times ((\theta_{b1} - \theta_{b2}))}{T_b(\theta'_{b1} - \theta'_{b2}) - T'_b(\theta_{b1} - \theta_{b2})}$$

## Fig. 16

$\Theta_{conducteur}$        $T_1$        $\Theta_{b1}$        $T_b$        $\Theta_{b2}$

$W_c$

## Fig. 17

$\Theta_{conducteur}$        $T_1$        $\Theta_{b1}'$        $T_b'$        $\Theta_{b2}'$

$W_c$

## Fig. 18

$$\theta_{conducteur} = \frac{\theta'_{b1} - \frac{B}{A}\,\theta_{b1}}{1 - \frac{B}{A}}$$

## Fig. 19

Europäisches
Patentamt

European
Patent Office

Office européen
des brevets

## RAPPORT DE RECHERCHE EUROPEENNE

**Numéro de la demande**

EP 25 15 9524

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | PSOMOPOULOS C.S. ET AL: "Electricity savings and CO2 emissions reduction in buildings sector: How important the network losses are in the calculation?", ENERGY, vol. 35, no. 1, 1 janvier 2010 (2010-01-01), pages 485-490, XP093200948, AMSTERDAM, NL ISSN: 0360-5442, DOI: 10.1016/j.energy.2009.10.016 * le document en entier * ----- | 1-18 | INV. G01N25/18 G01N33/00 G01R21/02 G01K3/14 G01K7/42 G01R21/14 G01R31/08 |
| X | Baldasano José María ET AL: "Estimate of energy consumption and CO 2 emission associated with the production, use and final disposal of PVC, XLPE and PE cables", , 30 novembre 2005 (2005-11-30), XP093288666, Extrait de l'Internet: URL:https://www.pvcforum.it/pvc_library/18 -LCA%20-%20EPD/UPC%20-%20Cables%20final%20 report.pdf [extrait le 2025-06-20] * abrégé * * pages 4-16 * * pages 19-23 * * tableaux 4.5, 5.1-5.5 * ----- | 1-18 | **DOMAINES TECHNIQUES RECHERCHES (IPC)** G01R G01N G01K H02J |
| A | WO 2012/127601 A1 (PASCO CORP [JP]; UCHIMA MITSUAKI [JP] ET AL.) 27 septembre 2012 (2012-09-27) * le document en entier * ----- -/-- | 1-18 | |

Le présent rapport a été établi pour toutes les revendications

| Lieu de la recherche | Date d'achèvement de la recherche | Examinateur |
|---|---|---|
| La Haye | 27 juin 2025 | Totò, Nicola |

EPO FORM 1503 03.82 (P04C02)

page 1 de 2

Europäisches
Patentamt

European
Patent Office

Office européen
des brevets

# RAPPORT DE RECHERCHE EUROPEENNE

Numéro de la demande

EP 25 15 9524

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | VORSIC ZIGA ET AL: "New Method for Calculating the Heating of the Conductor", ENERGIES, vol. 12, no. 14, 18 juillet 2019 (2019-07-18), page 2769, XP093201169, CH ISSN: 1996-1073, DOI: 10.3390/en12142769 ----- | 1-18 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| La Haye | 27 juin 2025 | Totò, Nicola |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
..................................................................................................
& : membre de la même famille, document correspondant

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

EP 25 15 9524

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-06-2025

| Document brevet cité au rapport de recherche | Date de publication | Membre(s) de la famille de brevet(s) | Date de publication |
|---|---|---|---|
| WO 2012127601 A1 | 27-09-2012 | AUCUN | |

Pour tout renseignement concernant cette annexe : voir Journal Officiel de l'Office européen des brevets, No.12/82